Europäisches Patentamt

European Patent Office

Office européen des brevets

(11) Publication number: **0 313 402 B1**

# EUROPEAN PATENT SPECIFICATION

(45) Date of publication of patent specification: **20.05.92**  (51) Int. Cl.⁵: **A01G  7/00**

(21) Application number: **88309952.5**

(22) Date of filing: **21.10.88**

(54) **Method for culturing plant tissue.**

(30) Priority: **23.10.87 JP 266519/87**

(43) Date of publication of application:
**26.04.89 Bulletin  89/17**

(45) Publication of the grant of the patent:
**20.05.92 Bulletin  92/21**

(84) Designated Contracting States:
**AT BE CH DE ES FR GB GR IT LI LU NL SE**

(56) References cited:
**EP-A- 0 276 575
EP-A- 0 281 374
WO-A-87/02701**

(73) Proprietor: **MITSUI PETROCHEMICAL INDUS-
TRIES, LTD.
2-5, Kasumigaseki 3-chome Chiyoda-ku
Tokyo 100(JP)**

(72) Inventor: **Shizufumi, Tanimoto
2-6, Misono 1-chome
Otake-shi Hiroshima, 739-06(JP)**
Inventor: **Shigeru, Takahashi
2-7, Misono 1-chome
Otake-shi Hiroshima, 739-06(JP)**

(74) Representative: **Myerscough, Philip Boyd et al
J.A.Kemp & Co. 14, South Square Gray's Inn
London, WC1R 5EU(GB)**

## Description

This invention relates to a method for culturing a plant tissue, particularly to a method suitable for propagation of plants in large quantities.

Vegetables such as cabbage, tomato, potato, asparagus, carrot, soybean, onion, and rice are utilized as foods, and tulip, torenia, Easter lily, cornflower and rudbeckia are beloved as ornamental garden plants. The proliferation of these plants has so far carried out by seeding, bulb splitting or tuber splitting. However, these proliferation methods not only require many hands and lands but also have problems with reduced growth rate of seeds and seedlings and the quality of flowers because of the spread of viral diseases. In addition, in order to raise and maintain a variety having favorable characters, it is effective to let a plant make vegetative propagation. Recently, there have been reports on a method utilizing plant tissue culture techniques for the purpose of improving these problems and proliferation efficiency (e.g. JP-A-15734/1980). It has been considered that the proliferation according to the tissue culture techniques is accomplished through the differentiation of an adventitious bud, an adventitious embryo or a bulb from a cultured tissue section or a cultured cell and that the differentiation is controlled by the concentration ratio of auxin and cytokinin as plant hormones (e.g., Annals of Botany, vol. 45, pp. 321-327, 1980). However, there are numerous plant species which are not differentiated only by the plant hormones and, even though differentiation takes place, there are also plant species which are poorly differentiated, so that a more direct and effective differentiation-inducing method is expected to be established.

The invention provides a method of culturing a plant tissue, characterised by culturing tissue sections or cultured cells of a plant using a medium comprising a phospholipid.

The phospholipid accelerates the differentiation of an adventitious bud, an adventitious embryo and a bulb of a plant by mobilizing calcium ions in a cell.

The method of the invention enables a plant body of high quality to be cultured efficiently in large quantities from tissue sections or cultured cells of a plant as compared with the conventional method, so that plants can be propagated in large quantities.

Prior to this invention, we found that the differentiation of a plant cell into an adventitious bud or an adventitious embryo was accelerated when the tissue culture was carried out by introducing calcium ions into a plant cell from outside the cell (see EP-A-276575 published 3 August 1988 and EP-A-281374 published 7 September, 1988). In accordance with the present invention it has been found that the differentiation of a plant cell could be accelerated when the plant tissue culture was carried out using a phospholipid. The phospholipid moves calcium ions originally localized and accumulated at a specific organelle in a cell into a cytoplasm to activate the calcium even when tissue culture is carried out without adding a calcium ion to a medium to introduce the calcium into a cell from the outside.

In the following description, the movement of calcium into a cytoplasm will be expressed as "calcium ions in a cell are mobilized" for convenience.

There is no particular restriction on plants to which the tissue culture method according to the present invention can be applied. That is, the present method can be applied to all plants.

As examples of a plant to which the present method is applicable, plants belonging to the orders *Solanales, Papaverales, Umbellales, Rosalesm, Liliales, Asterales, Geraniales* and *Graminales* can be enumerated. As specific examples of these plants, the plants described in "*Fundamentals of Classification of Plant System*" [T. Yamagishi (ed.), Hokuryukan, (1974)] can be enumerated. As more specific examples thereof, egg plant, tomato, potato and tobacco as plants belonging to the order *Solanales*; cabbage and Japanese radish as plants belonging to the order *Papaverales*; carrot and parsley as plants belonging to the order *Umbellales*; rose, strawberry and soybean as plants belonging to the order *Rosales*; onion, tulip and Easter lily as plants belonging to the order *Liliales*; chrysanthemum, cornflower and sunflower as plants belonging to the order *Asterales*; crane's-bill and flax as plants belonging to the order *Geraniales* and rice and corn as plants belonging to the order *Graminales* can be enumerated. Among these plants, tomato, egg plant, torenia, carrot, cabbage, onion, perilla, soybean, cornflower, rudbeckia, Easter lily, tulip, asparagus, flax and rice can be enumerated as specific examples of favorable plants.

In the present invention, the tissue culture of a plant can be carried out by using a tissue secton or a cultured cell of a plant.

As specific examples of the tissue section, plant tissue sections to be obtained by cutting a cotyledon, a hypocotyl, a shoot apex, a stem, a leaf, a scale, a root or other tissues into pieces can be enumerated. These tissue sections are generally collected and used after sterilizing the concerned plant with sodium hypochlorite and ethyl alcohol. However, in case of using a sterilely cultivated plant, the above sterilization step is not required. In case of propagation of plants having no disease or virus, tissues in the neighborhood of apical meristems, the foregoing tissue sections of a plant obtained from tissues in the neighborhood of apical meristems, etc. can be used as culture materials. Cultured cells which can be used for the plant tissue culture according to the present invention are undifferentiated protean cells including callus tissues which can be obtained by culturing the foregoing tissue sections according to the publicly known methods. In forming plants by culturing tissue sections or cultured cells of a plant, a method to be described in detail below is adopted in the present invention.

In the tissue culture method according to the present invention, a method in which tissue sections or cultured cell of a plant is cultured by using a medium containing a phospholipid as a substance mobilizing calcium ions in a cell is employed. The phospholipid can be arachidonic acid, a prostaglandin or a glycerophospholipid for example, and it is particularly preferable to use glycerophospholipid. Specific examples of the glycerophospholipid include phosphatidic acid, lysophosphatidic acid, phosphatidylinositol, phosphatidylcholine, phosphatidylethanolamine, phosphatidylserine and phosphatidylglycerol, and diolein as a synthetic phospholipid. The present invention employs a tissue culture method using a medium containing at least one phospholipid. According to the method, the differentiation of tissue sections or cultured cells of a plant into adventitious buds, adventitious embryos and bulbs is strikingly accelerated.

In the present invention, a medium to be used by adding thereto the phospholipid such as phosphatidic acid is a medium containing inorganic constituents and a carbon source as essential constituents, plant hormones, vitamins and, on demand, amino acids. As inorganic constituents of the medium, inorganic salts containing elements such as nitrogen, phosphorous, potassium, sodium, calcium, magnesium, sulfur, iron, manganese, zinc, boron, molybdenum, chlorine, iodine, cobalt and copper can be enumerated. Specifically, compounds such as potassium nitrate, sodium nitrate, ammonium nitrate, ammonium chloride, monbasic potassium phosphate, dibasic sodium phosphate, magnesium sulfate, magnesium chloride, sodium sulfate, ferrous sulfate, ferric sulfate, manganese sulfate, copper sulfate, ammonium molybdate, molybdenum trioxide, sodium molybdate, potassium iodide, zinc sulfate, boric acid and cobalt chloride can be exemplified.

As carbon sources of the medium, carbohydrate such as sucrose and its derivative, organic acid such as fatty acid, primary alcohol such as ethanol can be exemplified.

As plant hormones of the medium, auxins such as 1-naphthaleneacetic acid (NAA), 3-indoleacetic acid (IAA), p-chlorophenoxyaceticacid, 2,4-dichlorophenoxyacetic acid (2,4-D), 3-indolebutyric acid (IBA), their derivatives, etc. and cytokinins such as 6-benzyladenine (BA), kinetine and zeatin can be exemplified.

As vitamins of the medium, biotin, thiamine (vitamin $B_1$), pyridoxine (vitamin $B_6$) , calcium pantothenate, ascorbic acid (vitamin C), inositol, nicotinic acid, nicotinamide and riboflavin (vitamin $B_2$) can be exemplified.

As amino acids of the medium, glycine, alanine, glutamic acid, cystine, phenylalanine, proline and lysine can be exemplified.

It is preferred that the foregoing medium of the present invention is used by adding thereto the foregoing constituents at the following concentrations: inorganic constituents, approx. 0.1$\mu$M to approx. 100mM; carbon sources, approx. 1 to approx. 100g/$\ell$; plant hormones, 0.01 to approx. 10mg/$\ell$; vitamins, approx. 0.1 to approx. 150mg/$\ell$; and amino acids, 0 to approx. 1,000mg/$\ell$.

As specific examples of the foregoing medium to be used in the present tissue culture, media prepared by adding the foregoing carbon sources, plant hormones and, on demand, the foregoing vitamins and amino acids to the conventionally known media for the plant tissue culture such as Murashige-Skoog's medium ('62), White's medium ('63), Gamborg's B-5 medium, Mitsui's M-9 medium and Nitsch-Nitsch's medium can be enumerated. Among these, it is particularly preferable to use a medium prepared by employing Nitsch-Nitsch's medium, Linsmaier-Skoog's medium or Murashige-Skoog's medium. Incidentally, the composition of the above conventionally known media are described, for example, in "*Tissue Culture of Plant Cell*" [H. Harada and A. Komamine, pp. 390 ~ 391, Rikougakusha (1979)].

The foregoing medium which can be used in the present invention is a liquid medium or a solid medium containing generally 0.2 to 1.5% agar or 0.2 to 0.4% gellite.

In the present invention, the concentration of phospholipid to be added to the foregoing medium in the medium is generally in the range of $10^{-9}$ to $10^{-5}$M and preferably in the range of $10^{-7}$ to $10^{-5}$M.

In the present invention, the foregoing tissue sections or cultured cells of a plant can be cultured also by using a liquid medium to which oxygen-containing gas is gassed as described in JP-A-285928/1986.

According to the present method, an adventitious bud, an adventitious embryo or a daughter bulb (small bulb) can be obtained from tissue sections or cultured cells of a plant efficiently in large quantities. Furthermore, an adventitious bud and an adventitious embryo obtained according to the present method are rooted to form a plant body and then cut into tissue sections. A daughter bulb obtained according to the present method can also be cut into tissue sections. These tissue sections are further cultured according to the foregoing present tissue culture method, whereby seeds and seedlings can be proliferated in large quantities. Incidentally, the plants obtained according to the present invention can be grown into sound plant bodies having uniform characters by an ordinary cultivation.

The present invention will now be described by reference to Examples and Comparative Examples.

Examples 1 to 8

A scale was collected from a bulb of a sterilely raised Easter lily and cut into 2mm wide sections. On the other hand, a sterile Murashige-Skoog's solid medium (1962) of pH 5.7 (gellite concentration: 0.2%) containing 4% sucrose, $10^{-7}$M 1-naphthaleneacetic acid, $10^{-7}$M 6-benzyladenine and, as phospholipid, a compound selected from phosphatidic acid, lysophosphatidic acid, phosphatidylinositol, phosphatidylethanolamine, phosphatidycholine, phosphatidylserine, phosphatidylglycerol and diolein was prepared. The Easter lily scale sections prepared above were placed in the above medium and cultured at 25°C for 5 weeks in a light place. The resulting number of bulb differentiated per scale in each treated sample is given in Table 1.

In any of the treated samples, the number of bulb obtained by the differentiation was increased, as compared with that of the following Comparative Example 1.

Comparative Example 1

The tissue culture of Easter lily scale sections was carried out in the same manner as in Example 1, except that a medium not containing the phospholipid was used. The results are given in Table 1.

Examples 9 to 16

The tissue culture of Easter lily scale sections was carried out in the same manner as in Examples 1 to 8, except that a Murashige-Skoog's medium not containing calcium was used and that 0.8mM ethylene glycolaminoethyl ether tetraacetic acid was added to the medium in order to remove calcium out of cells. The results are given in Table 2.

After 1 week of culturing, the sections stained with Queen 2 as a calcium ion-specific fluorescent reagent to measure calcium ion concentrations in cells by the use of a microscopic photometer. The results are given in Table 2.

From Table 2, it is clear that, although calcium ions were not contained in a medium, the calcium ions in cells were mobilized by adding phospholipid to the medium to accelerate the differentiation.

Comparative Example 2

Easter lily scale sections were cultured in the same manner as in Example 9, except that a medium not containing the phospholipid was used. The results are given in Table 2.

Examples 17 to 35

The tissue culture was carried out in the same manner as in Example 1, except that tomato callus cells, torenia hypocotyl sections, asparagus stem sections, egg plant hypocotyl sections, perilla leaf sections, cornflower hypocotyl sections, rudbeckia leaf sections and flax stem sections were used as materials. The results are given in Table 3.

In any of the treated samples, the mumber of adventitious bud differentiated was increased, as compared with those of the following Comparative Examples 3 to 10.

Comparative Examples 3 to 10

The tissue culture of tomato callus cells, torenia hypocotyl sections, asparagus stem sections, egg plant hypocotyl sections, perilla leaf sections, cornflower hypocotyl sections, rudbeckia leaf sections and flax stem sections was carried out in the same manner as in Examples 17 to 35, except that a medium not containing the phospholipid was used. The results are given in Table 3.

4

Examples 36 to 42

The tissue culture was carried out in the same manner as in Example 1, except that cabbage callus cells, soybean callus cells, rice callus cells and carrot hypocotyl sections were used as materials. The results are given in Table 4.

In any of the treated samples, the number of adventitious bud differentiated was increased, as compared with those of the following Comparative Examples 11 to 14.

Comparative Examples 11 to 14

The tissue culture of cabbage callus cells, soybean callus cells, rice callus cells and carrot hypocotyl sections was carried out in the same manner as in Examples 36 to 42, except that a medium not containing the phospholipid was used. The results are given in Table 4.

Examples 43 to 46

The tissue culture was carried out in the same manner as in Example 1, except that onion scale sections and tulip scale sections were used as materials. The results are given in Table 5.

In any of the treated samples, the number of bulb differentiated was increased, as compared with those of the following Examples 15 and 16.

Comparative Examples 15 and 16

The tissue culture of onion scale sections and tulip scale sections was carried out in the same manner as in Examples 43 to 46, except that a medium not containing the phospholipid was used. The results are given in Table 5.

Table 1

| Example No. | Treatment | Concentration | No. of bulb differentiated/section |
|---|---|---|---|
| Comparative Example 1 | No addition | - | 2.1 |
| Example 1 | Phosphatidic acid | $10^{-6}$ M | 6.8 |
| Example 2 | Lysophosphatidic acid | $10^{-6}$ M | 5.8 |
| Example 3 | Phosphatidylinositol | $10^{-7}$ M | 7.2 |
| Example 4 | Phosphatidylethanolamine | $10^{-7}$ M | 4.6 |
| Example 5 | Phosphatidylcholine | $10^{-7}$ M | 4.4 |
| Example 6 | Phosphatidylserine | $10^{-5}$ M | 6.7 |
| Example 7 | Phosphatidylglycerol | $10^{-7}$ M | 7.6 |
| Example 8 | Diolein | $10^{-6}$ M | 8.2 |

T a b l e  2

| Example No. | Treatment | Concentration | $Ca^{2+}$ Concentration in Cell | No. of bulb differentiated/section |
|---|---|---|---|---|
| Comparative Example 2 | No addition | — | $0.02\,\mu M$ | 1.8 |
| Example 9 | Phosphatidic acid | $10^{-6}M$ | $0.9\,\mu M$ | 8.2 |
| Example 10 | Lysophosphatidic acid | $10^{-6}M$ | $0.8\,\mu M$ | 7.2 |
| Example 11 | Phosphatidylinositol | $10^{-7}M$ | $1.2\,\mu M$ | 8.6 |
| Example 12 | Phosphatidylethanolamine | $10^{-7}M$ | $0.8\,\mu M$ | 5.8 |
| Example 13 | Phosphatidylcholine | $10^{-7}M$ | $0.7\,\mu M$ | 5.2 |
| Example 14 | Phosphatidylserine | $10^{-5}M$ | $0.9\,\mu M$ | 8.0 |
| Example 15 | Phosphatidylglycerol | $10^{-7}M$ | $1.0\,\mu M$ | 8.4 |
| Example 16 | Diolein | $10^{-6}M$ | $1.4\,\mu M$ | 8.8 |

EP 0 313 402 B1

T a b l e  3-1

| Example No. | Material | Treatment | Concentration | Number of Adventitious Bud Differentiated/Section |
|---|---|---|---|---|
| Comparative Example 3 | Tomato callus cell | No addition | — | 2.2 |
| Comparative Example 4 | Torenia hypocotyl section | No addition | — | 6.5 |
| Comparative Example 5 | Asparagus stem section | No addition | — | 0.4 |
| Comparative Example 6 | Egg plant hypocotyl section | No addition | — | 1.8 |
| Comparative Example 7 | Perilla leaf section | No addition | — | 2.8 |
| Comparative Example 8 | Cornflower hypocotyl section | No addition | — | 2.0 |
| Comparative Example 9 | Rudbeckia leaf section | No addition | — | 1.8 |
| Comparative Example 10 | Flax stem section | No addition | — | 5.6 |
| Example 17 | Tomato callus cell | Phosphatidic acid | $10^{-6}M$ | 5.4 |
| Example 18 | Tomato callus cell | Phosphatidylinositol | $10^{-7}M$ | 6.2 |
| Example 19 | Tomato callus cell | Phosphatidylglycerol | $10^{-7}M$ | 6.4 |

Table 3-2

| Example No. | Material | Treatment | Concentration | Number of Adventitious Bud Differentiated/Section |
|---|---|---|---|---|
| Example 20 | Torenia hypocotyl section | Phosphatidic acid | $10^{-8}$M | 24.8 |
| Example 21 | Torenia hypocotyl section | Lysophosphatidic acid | $10^{-6}$M | 20.2 |
| Example 22 | Torenia hypocotyl section | Phosphatidylserine | $10^{-5}$M | 26.6 |
| Example 23 | Asparagus stem section | Phosphatidic acid | $10^{-6}$M | 4.2 |
| Example 24 | Asparagus stem section | Phosphatidylcholine | $10^{-7}$M | 3.0 |
| Example 25 | Asparagus stem section | Phosphatidylethanol-amine | $10^{-6}$M | 2.6 |
| Example 26 | Egg plant hypocotyl section | Phosphatidic acid | $10^{-6}$M | 3.4 |
| Example 27 | Egg plant hypocotyl section | Lysophosphatidic acid | $10^{-6}$M | 2.8 |
| Example 28 | Egg plant hypocotyl section | Phosphatidylinositol | $10^{-7}$M | 4.2 |
| Example 29 | Perilla leaf section | Phosphatidic acid | $10^{-6}$M | 6.6 |

EP 0 313 402 B1

T a b l e   3-3

| Example No. | Material | Treatment | Concentration | Number of Adventitious Bud Differentiated/Section |
|---|---|---|---|---|
| Example 30 | Perilla leaf section | Phosphatidylglycerol | $10^{-7}M$ | 7.4 |
| Example 31 | Perilla leaf section | Phosphatidylserine | $10^{-5}M$ | 7.2 |
| Example 32 | Cornflower hypocotyl section | Phosphatidic acid | $10^{-6}M$ | 8.6 |
| Example 33 | Cornflower hypocotyl section | Phosphatidylinositol | $10^{-7}M$ | 9.0 |
| Example 34 | Rudbeckia leaf section | Phosphatidic acid | $10^{-6}M$ | 4.6 |
| Example 35 | Flax stem section | Phosphatidic acid | $10^{-6}M$ | 12.8 |

EP 0 313 402 B1

Table 4

| Example No. | Material | Treatment | Concentration | Number of Adventitious embryo Formed/Section |
|---|---|---|---|---|
| Comparative Example 11 | Cabbage callus cell | No addition | — | 0 |
| Comparative Example 12 | Soybean callus cell | No addition | — | 0 |
| Comparative Example 13 | Rice callus cell | No addition | — | 0.2 |
| Comparative Example 14 | Carrot hypocotyl section | No addition | — | 0.2 |
| Example 36 | Cabbage callus cell | Phosphatidic acid | $10^{-6}$M | 1.2 |
| Example 37 | Cabbage callus cell | Phosphatidylinositol | $10^{-7}$M | 1.4 |
| Example 38 | Soybean callus cell | Phosphatidic acid | $10^{-6}$M | 0.8 |
| Example 39 | Soybean callus cell | Phosphatidylglycerol | $10^{-7}$M | 1.2 |
| Example 40 | Rice callus cell | Phosphatidic acid | $10^{-6}$M | 1.6 |
| Example 41 | Carrot hypocotyl section | Phosphatidic acid | $10^{-6}$M | 1.2 |
| Example 42 | Carrot hypocotyl section | Phosphatidylglycerol | $10^{-7}$M | 0.8 |

**Claims**

1. A method of culturing a plant tissue, characterised by culturing tissue sections or cultured cells of a plant using a medium comprising a phospholipid.

2. A method according to claim 1 wherein the phospholipid is arachidonic acid, a prostaglandin or a glycerophospholipid.

T a b l e  5

| Example No. | Material | Treatment | Concentration | Number of Bulb Formed/Section |
|---|---|---|---|---|
| Comparative Example 15 | Onion scale section | No addition | — | 3.8 |
| Comparative Example 16 | Tulip scale section | No addition | — | 2.8 |
| Example 43 | Onion scale section | Phosphatidic acid | $10^{-6}$M | 8.4 |
| Example 44 | Onion scale section | Phosphatidylglycerol | $10^{-6}$M | 7.8 |
| Example 45 | Tulip scale section | Phosphatidic acid | $10^{-6}$M | 6.0 |
| Example 46 | Tulip scale section | Phosphatidylserine | $10^{-5}$M | 5.6 |

3. A method according to claim 2, wherein the glycerophospholipid is phosphatidic acid, lysophosphatidic acid, phosphatidylinositol, phosphatidylserine, phosphatidylethanolamine, phosphatidylcholine or phosphatidylglycerol.

4. A method according to any one of claims 1 to 3 wherein the phospholipid is present in the medium at a concentration of from $10^{-9}$ M to $10^{-5}$ M.

5. A method according to claim 4 wherein the phospholipid is present in the medium at a concentration of from $10^{-7}$ M to $10^{-5}$ M.

6. A method according to any one of the preceding claims wherein the medium comprises one or more inorganic constituents, a carbon source and plant hormones.

7. A method according to claim 6 wherein the medium comprises vitamins and, optionally, amino acids.

8. A method according to any one of the preceding claims wherein the medium is liquid or solid and comprises agar or gellite.

9. A method according to any one of the preceding claims wherein the tissue section is obtained from a cotyledon, a hypocotyl, a shoot apex, a stem, a leaf, a scale or a root.

**Revendications**

1. Procédé de culture d'un tissu végétal, caractérisé par la culture de fragments de tissus ou de cellules cultivées d'une plante, au moyen d'un milieu comprenant un phospholipide.

2. Procédé selon la revendication 1, dans lequel le phospholipide est l'acide arachidonique, une prostaglandine ou un glycérophospholipide.

3. Procédé selon la revendication 2, dans lequel le glycérophospholipide est un acide phosphatidique, un acide lysophosphatidique, un phosphatidylinositol, une phosphatidylsérine, une phosphatidyléthanolamine, une phosphatidylcholine ou un phosphatidylglycérol.

4. Procédé selon l'une quelconque des revendications 1 à 3, dans lequel le phospholipide est présent dans le milieu à une concentration de $10^{-9}$ à $10^{-5}$ M.

5. Procédé selon la revendication 4, dans lequel le phospholipide est présent dans le milieu à une concentration de $10^{-7}$ à $10^{-5}$ M.

6. Procédé selon l'une quelconque des revendications précédentes, dans lequel le milieu comprend un ou plusieurs constituants minéraux, une source de carbone et des hormones végétales.

7. Procédé selon la revendication 6, dans lequel le milieu comprend des vitamines et éventuellement des aminoacides.

8. Procédé selon l'une quelconque des revendications précédentes, dans lequel le milieu est liquide ou solide et comprend de la gélose ou de la gellite.

9. Procédé selon l'une quelconque des revendications précédentes, dans lequel le fragment de tissu est obtenu à partir d'un cotylédon, d'un axe hypocotylé, d'un apex de pousse, d'une tige, d'une feuille, d'une écaille ou d'une racine.

**Patentansprüche**

1. Verfahren zur Kultivierung eines Pflanzengewebes, gekennzeichnet durch Kultivierung von Gewebeabschnitten oder kultivierten Zellen einer Pflanze unter Verwendung eines Mediums, das ein Phospholipid enthält.

2. Verfahren nach Anspruch 1, in dem das Phospholipid Arachidonsäure, ein Prostaglandin oder ein Glycerophospholipid ist.

3. Verfahren nach Anspruch 2, in dem das Glycerophospholipid Phosphatidsäure, Lysophosphatidsäure, Phosphatidylinositol, Phosphatidylserin, Phosphatidylethanolamin, Phosphatidylcholin oder Phosphatidylglycerin ist.

4. Verfahren nach einem der Ansprüche 1 bis 3, in dem das Phospholipid im Medium in einer Konzentration von $10^{-9}$ M bis $10^{-5}$ M vorliegt.

5. Verfahren nach Anspruch 4, in dem das Phospholipid im Medium in einer Konzentration von $10^{-7}$ M bis $19^{-5}$ M vorliegt.

6. Verfahren nach einem der vorstehenden Ansprüche, in dem das Medium ein oder mehrere anorganische Bestandteile, einen Kohlenstofflieferanten und Pflanzenhormone enthält.

7. Verfahren nach Anspruch 6, in dem das Medium Vitamine und gegebenenfalls Aminosäuren enthält.

8. Verfahren nach einem der vorstehenden Ansprüche, in dem das Medium flüssig oder fest ist und Agar oder Gellit enthält.

9. Verfahren nach einem der vorstehenden Ansprüche, in dem der Gewebeabschnitt von einem Kotyledon, Hypocotyl, einem Trieb, einem Stengel, einem Blatt, einer Schale oder einer Wurzel erhalten worden ist.